(19) Europäisches Patentamt
European Patent Office
Office européen des brevets

(11) Veröffentlichungsnummer : **0 171 728**
**B1**

(12) # EUROPÄISCHE PATENTSCHRIFT

(45) Veröffentlichungstag der Patentschrift : **05.04.89**

(51) Int. Cl.⁴ : **C 07 D401/06**, C 07 D403/06, A 61 K 31/40, A 61 K 31/445

(21) Anmeldenummer : **85109808.7**

(22) Anmeldetag : **05.08.85**

(54) **Neue Tryptamin-Derivate, ein Verfahren zu ihrer Herstellung und ihre Verwendung.**

(30) Priorität : **17.08.84 DE 3430284**

(43) Veröffentlichungstag der Anmeldung : **19.02.86 Patentblatt 86/08**

(45) Bekanntmachung des Hinweises auf die Patenterteilung : **05.04.89 Patentblatt 89/14**

(84) Benannte Vertragsstaaten : **AT BE CH DE FR GB IT LI NL SE**

(56) Entgegenhaltungen :

**CHEMICAL ABSTRACTS, Band 91, Nr. 21, 19. November 1979, Columbus, Ohio, USA, R. FUKUMORI; A. MINEGESHI; T. SATOH; H. KITAGAWA; S. YANAURA "Effect of disulfiram on turnover of 5-hydroxy-tryptamine in rat brain", p. 38, Zusammenfassung Nr. 168 362x**

**CHEMICAL ABSTRACTS, Band 94, Nr. 7, 16. Februar 1981, Columbus, Ohio, USA, J.C. AGARWAL; M. SHARMA; A.K. SAXENA; K. KISHOR; K.P. BHARGAVA; K. SHANKER "Synthesis and cardiovascular activity of piperidylethylindoles", p. 553, Zusammenfassung Nr. 47 062c**

**CHEMICAL ABSTRACTS, Band 94, Nr. 7, 16. Februar 1981, Columbus, Ohio, USA, C. GUEREMY; F. AUDIAU; A. CHAMPSEIX; A. UZAN; G. LE FUR; J. RATAUD "3-(4-Piperidinylalkyl)indoles, selective inhibitors of neuronal 5-hydroxy-tryptamine uptake", p. 13, Zusammenfassung Nr. 41 139m**

**CHEMICAL ABSTRACTS, Band 91, Nr. 11, 10. September 1979, Columbus, Ohio, USA, I. FLEMING; M. WOOLIAS "A new synthesis of indoles particularly suitable for the synthesis of tryptamines and tryptamine itself", p. 760, Zusammenfassung Nr. 91 438s**

(73) Patentinhaber : **Troponwerke GmbH & Co. KG**
**Berliner Strasse 156**
**D-5000 Köln 80 (DE)**

(72) Erfinder : **Glaser, Thomas, Dr.**
**Rybniker Strasse 6**
**D-5000 Köln 80 (DE)**
Erfinder : **Raddatz, Siegfried, Dr.**
**Jakob-Boehme-Strasse 21**
**D-5000 Köln 80 (DE)**
Erfinder : **Traber, Jörg, Dr.**
**Loewenburgstrasse 12**
**D-5204 Lohmar 21 (DE)**
Erfinder : **Allen, George, Prof. M.D.Vanderbilt University**
**Medical Center D-3213 Medical Center North**
**Nashville, TN 37232 (US)**

(74) Vertreter : **Mann, Volker, Dr. et al**
**c/o Bayer AG Konzernverwaltung RP Patentabteilung**
**D-5090 Leverkusen 1, Bayerwerk (DE)**

**EP 0 171 728 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft neue Tryptamine und deren Salze mit physiologisch unbedenklichen Säuren, ein Verfahren zu ihrer Herstellung sowie ihre Verwendung bei der Bekämpfung von Krankheiten.

Insbesondere betrifft die Erfindung die Verwendung dieser Substanzen zur Herstellung von Arzneimitteln zur Behandlung von Erkrankungen des zentralen Nervensystems durch eine Wirkung auf das serotoninerge System.

Aus CA 94, 1981, 47062c sind 1-Piperidinylethylindole bekannt, die in o-Position mit einem Alkylrest substituiert sind. Diese Verbindungen weisen eine blutdruckbeeinflussende Wirkung auf.

In CA 94, 1981, 41139m werden 4-Piperidinylalkylindole beschrieben, die eine Hemmung der Serotonin-Aufnahme in Synapsen und Blutplättchen bewirken.

Die Erfindung betrifft Tryptamine der allgemeinen Formel I

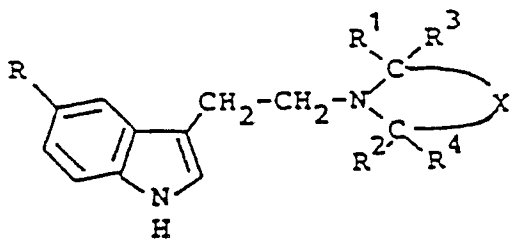

in welcher

R für Wasserstoff, Methyl, Fluor, Chlor, Brom, Cyano, Carbamoyl und Azido steht,
$R^1$ und $R^2$ gleich oder verschieden sind und Methyl oder Ethyl bedeuten,
$R^3$ und $R^4$ gleich oder verschieden sind und Wasserstoff, Methyl, Ethyl bedeuten, und
X für eine Alkylenkette mit 2 bis 4 Kohlenstoffatomen steht,

in der cis-Form, trans-Form und in Form jeglicher Gemische beider Isomere und die entsprechenden Salze.

Es wurde auch ein Verfahren zur Herstellung der erfindungsgemäßen Tryptamin-Derivate gefunden, das dadurch gekennzeichnet ist, daß man Indole der allgemeinen Formel II

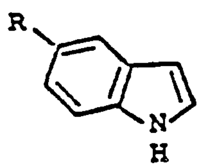

(II)

in welcher R die obengenannte Bedeutung hat, mit Oxalylchlorid zu Verbindungen der allgemeinen Formel IV

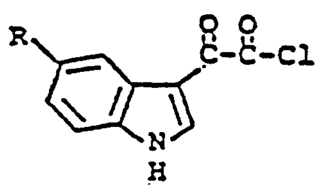

(IV)

in welcher R die obengenannte Bedeutung hat, umsetzt, die Verbindungen der Formel IV mit Aminen der Formel V

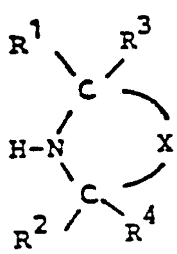

(V)

in welcher $R^1$, $R^2$, $R^3$, $R^4$ und X die obengenannte Bedeutung haben, zu Verbindungen der Formel VI

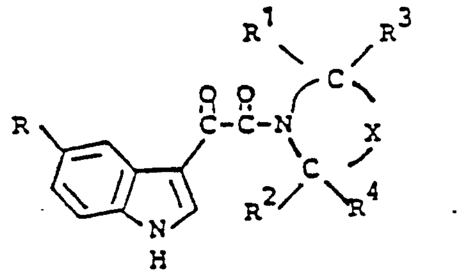

(VI)

in welcher R, $R^1$, $R^2$, $R^3$, $R^4$ und X die obengenannte Bedeutung haben, reagierenläßt und anschließend die Verbindungen der Formel VI reduziert.

Ähnliche Umsetzungsverfahren sind aus J. Am. Chem. Soc. 76, 6208 bis 8210 (1954) bekannt.

Die Ausgangsstoffe der Formeln II, III, VII und V sind handelsüblich und können nach bekannten Methoden hergestellt werden (J. Org. Chem. 41, 863 bis 869 (1976).

Das erfindungsgemäße Verfahren kann durch das folgende Reaktionsschema erläutert werden.

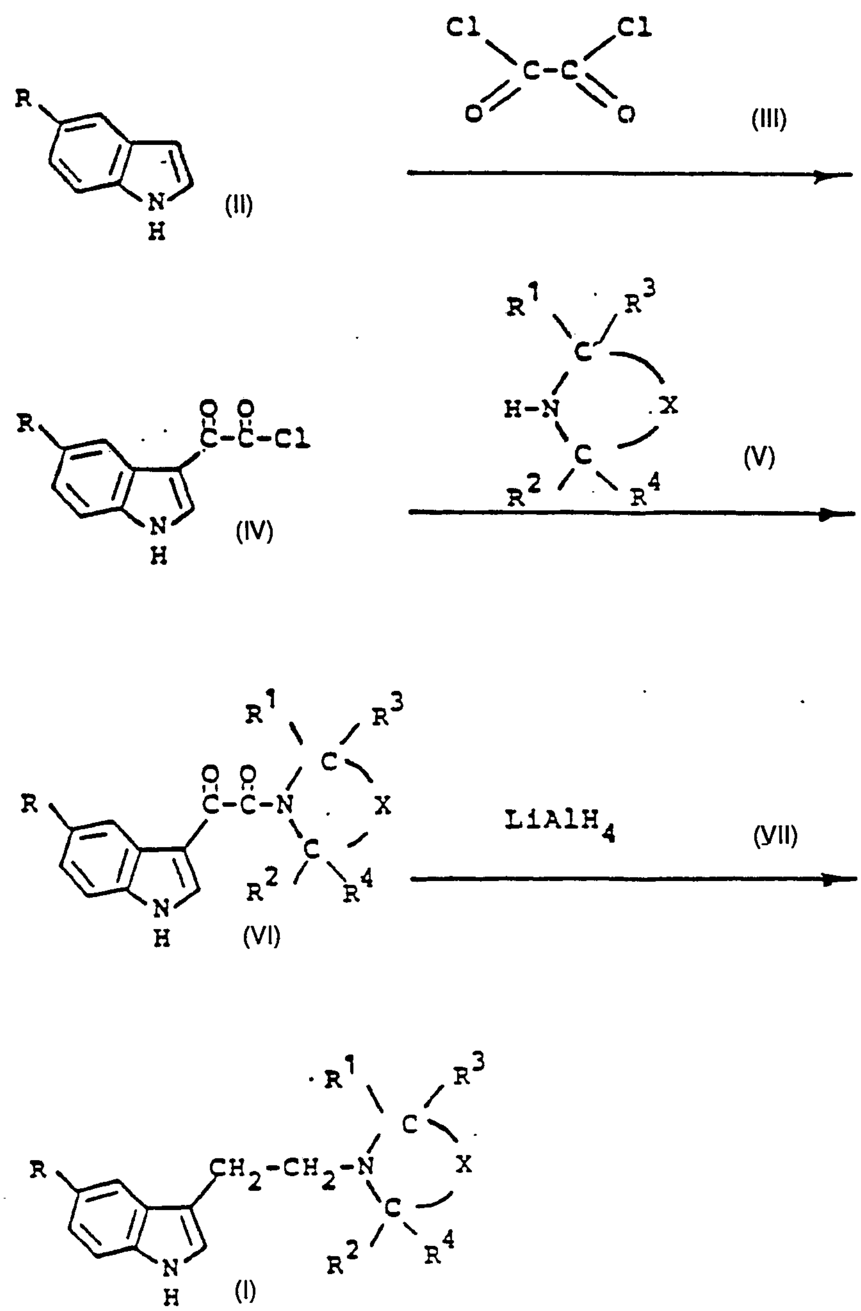


(II) (III) (IV) (V) (VI) (VII) (I)

Die 5-Cyanoverbindung läßt sich auch herstellen, indem man analog M.S. Newman, J. Org. Chem. 26, 1961, 2525 die 5-Bromverbindung in siedendem N-Methyl-2-pyrrolidon (202 °C) mit Kupfer(I)-cyanid reagieren läßt.

Die 5-Carbamoylverbindung wird hergestellt, indem man nach J. Hall, J. Org. Chem. 41, 1976, 3769 die 5-Cyanoverbindung mit Kaliumhydroxid in tert. Butanol ca. sieben Stunden unter Rückfluß erwärmt.

Die erfindungsgemäßen Tryptamin-Derivate unterscheiden sich von bekannten Tryptaminen dadurch, daß der basische Stickstoff sich in einem Ring befindet und durch Substituenten in unmittelbarer Nachbarschaft sterisch stark abgeschirmt ist. Es handelt sich hierbei um Verbindungen mit besonderer, eindeutig festgelegter Konformation, die es erlaubt, z. B. ganz bestimmte Subtypen von Serotoninrezeptoren des Gehirns zu besetzen, insbesondere solche des 5-$HT_1$-Typs (s. Tabelle).

5-$HT_1$-Rezeptoren sind in bestimmten Teilen des menschlichen Gehirns (J. M. Palacios et al., Brain Research 274, 1983, 150-155) und in Blutgefäßen des zentralen Nervensystems (S. J. Peroutka and M. J. Kuhar, Brain Research, 310, 1984, 193-196) nachgewiesen worden.

Die erfindungsgemäßen Verbindungen interagieren selektiv mit diesen 5-$HT_1$-Rezeptoren des Gehirns. Sie wirken in geeigneten Versuchsanordnungen antagonistisch, z. B. wird die durch Serotonin (5-Hydroxytryptamin = 5-HT) über 5-$HT_1$-Rezeptoren hervorgerufene Kontraktion der Arteria basilaris des Hundes (S. J. Peroutka et al., Brain Research 259, 1983, 327-330) durch die erfindungsgemäßen Wirkstoffe geblockt.

Auch wird die durch Serotonin bedingte Erhöhung der Adenylatcyclaseaktivität von neuralen NCB-20 Zellen (E. Berry-Kravis und G. Dawson, J. Neurochem. 41, 1983, 977-985) durch die erfindungsgemäßen Verbindungen verhindert.

Die Verbindungen sind somit selektive 5-$HT_1$-Antagonisten. Aufgrund dieser Eigenschaften muß man erwarten, daß die erfindungsgemäßen Verbindungen zur Behandlung von Erkrankungen des zentralen Nervensystems, insbesondere zur Behandlung von Schlafstörungen, Migräne, Vasospasmen und Ischämien geeignet sind. Sie stellen damit eine Bereicherung des Arzneimittelschatzes dar.

Erfindungsgemäß werden Tryptamin-Derivate der Formel (I) bevorzugt, worin

R für Wasserstoff, Fluor, Chlor oder Brom steht,

$R^1$ und $R^2$ gleich oder verschieden sind und Methyl oder Ethyl bedeuten,

$R^3$ und $R^4$ Wasserstoff bedeuten, und

X für eine Alkylenkette mit 3 Kohlenstoffatomen steht.

Die Verbindungen liegen in der cis-Form, in der trans-Form und in Form jeglicher Gemische beider Isomere vor.

Besonders bevorzugt werden Tryptamin-Derivate der Gruppe

3-[2-(cis- und trans-2,5-Dimethylpyrrolinyl)-ethyl]-indol-hydrochlorid,
3-[2-(cis-2,6-Dimethylpiperidinyl)-ethyl]-indol-hydrochlorid,
3-[2-(cis-2,6-Dimethylpiperidinyl)-ethyl]-5-bromindol-hydrochlorid,
3-[2-(cis-2,6-Dimethylpiperidinyl)-ethyl]-5-chlorindol-hydrochlorid und
3-[2-(cis-2,6-Dimethylpiperidinyl)-ethyl]-5-fluorindol-hydrochlorid.

Zur vorliegenden Erfindung gehören pharmazeutische Zubereitungen, die neben nicht-toxischen, inerten pharmazeutisch geeigneten Trägerstoffen eine oder mehrere der erfindungsgemäßen Verbindungen oder deren Salze enthalten, oder die aus einer oder mehrerer der erfindungsgemäßen Verbindungen oder deren Salzen bestehen, sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z. B. Tabletten, Dragees, Kapseln, Pillen, Suppositorien und Ampullen vorliegen, deren Wirkstoffgehalt einem Bruchteil oder einem Vielfachen einer Einzeldosis entspricht. Die Dosierungseinheiten können z. B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird, und die gewöhnlich einer ganzen, einer halben oder einem Drittel oder einem Viertel einer Tagesdosis entspricht.

Unter nicht-toxischen, inerten, pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen genannt.

Tabletten, Dragees, Kapseln, Pillen und Granulate können den oder die Wirkstoffe neben den üblichen Trägerstoffen enthalten, wie

a) Füll- und Streckmittel,
z. B. Stärke, Milchzucker, Rohrzucker, Glucose, Mannit und Kieselsäure,
b) Bindemittel,
z. B. Carboxymethylcellulose, Alginate, Gelatine, Polyvinylpyrrolidon,
c) Feuchthaltemittel,
z. B. Glycerin,

d) Sprengmittel,
z. B. Agar-Agar, Calciumcarbonat und Natriumbicarbonat,
e) Lösungsverzögerer,
z. B. Paraffin,
f) Resorptionsbeschleuniger,
z. B. quaternäre Ammoniumverbindungen,
g) Netzmittel,
z. B. Cetylalkohol, Glycerinmonostearat,
h) Adsorptionsmittel,
z. B. Kaolin und Bentonit und
i) Gleitmittel,
z. B. Talkum, Calcium- und Magnesiumstearat und feste Polyethylenglykole oder Gemische der unter a) bis i) aufgeführten Stoffe.

Die Tabletten, Dragees, Kapseln, Pillen und Granulate können mit den üblichen, gegebenenfalls Opalisierungsmittel enthaltenden Überzügen und Hüllen versehen sein und auch so zusammengesetzt sein, daß sie den oder die Wirkstoffe nur oder bevorzugt in einem bestimmten Teil des Intestinaltraktes, gegebenenfalls verzögert abgeben, wobei als Einbettungsmassen z. B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z. B. Polyethylenglykole, Fette, z. B. Kakaofett, und höhere Ester (z. B. $C_{14}$-Alkohol mit $C_{16}$-Fettsäure) oder Gemische dieser Stoffe.

Lösungen und Emulsionen können neben dem oder den Wirkstoffen die üblichen Trägerstoffe wie Lösungsmittel, Lösungsvermittler und Emulgator, z. B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl, Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe, wie flüssige Verdünnungsmittel, z. B. Wasser, Ethylalkohol, Propylenglykol, Suspendiermittel, z. B. ethoxylierte Isostearylalkohole, Polyoxyethylensorbit- und sorbitanester, mikrokristalline Cellulose, Aluminiummetahydroxid, Bentonit, Agar-Agar und Traganth oder Gemische dieser Stoffe enthalten.

Die genannten Formulierungsformen können auch Färbemittel, Konservierungsstoffe sowie geruchs- und geschmacksverbessernde Zusätze, z. B. Pfefferminzöl und Eukalyptusöl, und Süßmittel, z. B. Saccharin, enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-% der Gesamtmischung, vorhanden sein. Die oben aufgeführten pharmazeutischen Zubereitungen können außer Verbindungen der Formel I und/oder deren Salzen auch andere pharmazeutische Wirkstoffe enthalten.

Die Herstellung der oben aufgeführten pharmazeutischen Zubereitungen erfolgt in üblicher Weise nach bekannten Methoden, z. B. durch Mischen des oder der Wirkstoffe mit dem oder den Trägerstoffen.

Zur vorliegenden Erfindung gehören auch Verbindungen der Formel I und/oder deren Salze sowie pharmazeutische Zubereitungen, die eine oder mehrere Verbindungen der Formel I und/oder deren Salze enthalten, zur Verwendung in der Humanmedizin zur Verhütung, Besserung und/oder Heilung der oben aufgeführten Erkrankungen.

Die Wirkstoffe oder die pharmazeutischen Zubereitungen können vorzugsweise oral, parenteral und/oder rectal, vorzugsweise oral und parenteral, insbesondere oral und intravenös appliziert werden.

Im allgemeinen hat es sich als vorteilhaft erwiesen, den oder die Wirkstoffe bei parenteraler (i.v. oder i.m.) Applikation in Mengen von etwa 0,005 bis etwa 5, vorzugsweise von 0,01 bis 1 mg/kg Körpergewicht je 24 Stunden und bei oraler Applikation in Mengen von etwa 0,01 bis etwa 10, vorzugsweise 0,05 bis 5 mg/kg Körpergewicht je 24 Stunden gegebenenfalls in Form mehrerer Einzelgaben zur Erzielung der gewünschten Ergebnisse zu verabreichen. Eine Einzelgabe enthält den oder die Wirkstoffe vorzugsweise in Mengen von etwa 0,005 bis etwa 10, insbesondere 0,01 bis 1 mg/kg Körpergewicht.

Es kann jedoch erforderlich sein, von den genannten Dosierungen abzuweichen, und zwar in Abhängigkeit von der Art und dem Körpergewicht des zu behandelnden Objektes, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden

Fachmann aufgrund seines Fachwissens leicht erfolgen.

Zur vorliegenden Erfindung gehören auch solche Arzneimittel, die neben Verbindungen der Formel I noch weitere Wirkstoffe enthalten. Vorzugsweise seien genannt : β-Rezeptorenblocker, Parasympathikolytika, Anxiolytika, Neuroleptika, Hypnotika und Tranquilizer.

Beispiel 1

3-[2-(cis- und trans-2,5-Dimethylpyrrolidinyl)-ethyl]-indol-hydrochlorid

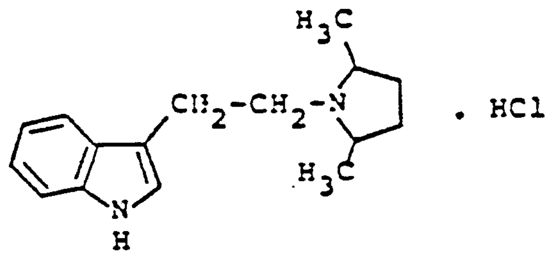

1.1.

Indolyl-3-glyoxylchlorid

Man löst 10 g (0,085 mol) Oxalylchlorid in Diethylether und tropft unter Rühren 11,1 g (0,088 mol) Indol in 25 ml Diethylether zu. Die Zugabe wird so dosiert, daß der Ether gerade siedet und die Chlorwasserstoffentwicklung nicht zu stark wird. Zur Vervollständigung der Reaktion erhitzt man noch zwei Stunden zum Sieden. Beim Abkühlen fällt ein farbloses (manchmal leicht grün gefärbtes) Kristallisat aus ; Schmelzpunkt : 138 °C.

Ausbeute : 15,7 g (89 % der Theorie).

1.2.

cis- und trans-Indolyl-3-glyoxyl-2,5-dimethylpyrrolidid

Man löst 10 g (0,048 mol) Indolyl-3-glyoxylchlorid in 200 ml Tetrahydrofuran (THF) und tropft unter Rühren eine Mischung von 4,9 g (0,049 mol) = 6,1 ml 2,5-Dimethylpyrrolidin (cis/trans-Gemisch) und 4,9 g Triethylamin (0,049 mol) = 6,7 ml in 100 ml THF zu. Sofort erfolgt Reaktion. Nach einer Stunde Nachreaktion engt man im Vakuum zur Trockne ein, nimmt den Rückstand in 100 ml Dichlormethan auf, schüttelt zweimal mit 50 ml Wasser aus, trocknet die organische Phase mit Natriumsulfat, engt auf ein kleines Volumen ein und chromatographiert (Kieselgel 60, Laufmittel : Dichlormethan/Essigsäureethylester = 4/1). Man erhält farblose Kristalle mit einem Schmelzpunkt von 140 °C.

Ausbeute : 10,3 g (80 % der Theorie).

1.3.

cis- und trans-3-[2-(2,5-Dimethylpyrrolidinyl)-ethyl]-indol-hydrochlorid

Man suspendiert 5,6 g (0,148 mol) Lithiumaluminiumhydrid in 400 ml THF und tropft unter Rühren bei Raumtemperatur 10 g (0,037 mol) cis- und trans-Indolyl-3-glyoxyl-2,5-dimethylpyrrolidid ein. Nach und nach tritt Gasentwicklung ein. Man vervollständigt die Reaktion, indem man noch fünf Stunden zum Sieden erhitzt.

Nach dem Abkühlen versetzt man vorsichtig unter Kühlung mit 5,6 ml Wasser, mit 4 ml 20 %iger Natronlauge und noch mit 18 ml Wasser. Den entstandenen Niederschlag filtriert man ab, rührt ihn mit ca. 50 ml THF aus und filtriert erneut. Die vereinigten Filtrate werden zur Trockne eingeengt und der Rückstand in 100 ml Methanol gelöst. Man versetzt mit ca. 50 ml 50 %-iger Salzsäure, engt im Vakuum zur Trockne ein und nimmt den Rückstand in 50 ml Wasser auf. Nach Zugabe von 20 ml 5 n Natronlauge (alkalisch stellen 1) schüttelt man dreimal mit 50 ml Dichlormethan aus. Die organischen Phasen werden vereinigt, mit Natriumsulfat getrocknet und nach Einengen auf ein kleines Volumen chromatographiert (Aluminiumoxid 90, Aktivität 2-3, Laufmittel : Dichlormethan/Essigsäureethylester = 4/1). Man erhält zwei Fraktionen. Die Lösungen werden zur Trockne eingedampft und die Rückstände aus Cyclohexan rekristallisiert. Man erhält jeweils farblose Kristalle. cis-Produkt :

Fp : = 139 °C, Ausbeute 4,8 g (53 % der Theorie)

trans-Produkt : Fp : = 154 °C, Ausbeute 3,0 g

(34 % der Theorie). Die Zuordnung der Konformationen erfolgt mit Hilfe der $^{13}C$-NMR-Spektroskopie.

Hydrochloride.

Man löst das jeweilige Amin in Dichlormethan/Isopropanol und versetzt mit 1 n Salzsäure. Nach Eindampfen zur Trockne rekristallisiert man aus Isopropanol/Diisopropylether = 1/1 und erhält jeweils farblose

Kristalle.

cis-Produkt : Fp = 220 °C (Zers.), Ausbeute 93 % d. Th.

trans-Produkt : Fp = 205 °C (Zers.), Ausbeute : 84 % d. Th.

## Beispiel 2

3-[2-(cis-2,6-Dimethylpiperidinyl)-ethyl]-indol-hydrochlorid

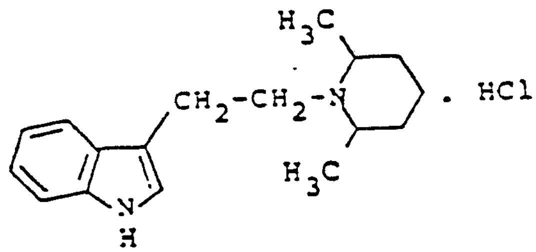

2.1.
Indolyl-3-glyoxylchlorid
analog 1.1. aus 111,7 g (0,88 mol) = 75 ml Oxalylchlorid und 100 g (0,853 mol) Indol
farblose Kristalle ; Schmelzpunkt : 138-9 °C
Ausbeute : 158,8 g (90 % der Theorie).
2.2.
cis-Indolyl-3-glyoxyl-2,6-dimethylpiperidid
analog 1.2. aus 10,4 g (0,05 mol) Indolyl-3-glyoxylchlorid und 10,1 g (0,1 mol) = 12,0 ml 2,6-cis-Dimethylpiperidin
farblose Kristalle ; Schmelzpunkt : 196 °C
Ausbeute : 12,5 g (46 % der Theorie).
2.3.
3-[2-(cis-2,6-Dimethylpiperidinyl)-ethyl]-indol-hydrochlorid
analog 1.3. aus 3,0 g (0,079 mol) Lithiumaluminiumhydrid und 5,5 g (0,02 mol) cis-Indolyl-3-glyoxyl-2,6-dimethylpiperidid, Chromatographie mit Kieselgel 60, Laufmittel : Methanol/Dichlormethan = 15/85 ; Rekristallisation aus Diisopropylether
farblose Kristalle ; Schmelzpunkt : 168 °C
Hydrochlorid
farblose Kristalle ; Schmelzpunkt ; 280-2 °C (Zers.)
Ausbeute : 4,7 g (81 % der Theorie).

## Beispiel 3

3-[2-(cis-2,6-Dimethylpiperidinyl)-ethyl]-5-bromindol-hydrochlorid

cis

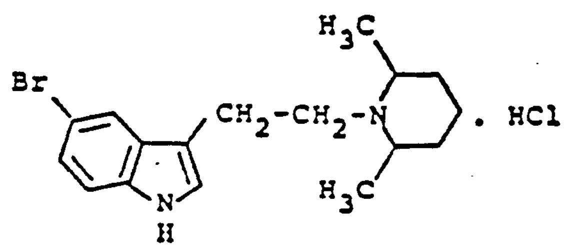

3.1.
cis-5-Bromindolyl-3-glyoxyl-2,6-dimethylpiperidid
In einem 500 ml-Dreihalskolben ($CaCl_2$-Rohr, Rückflußkühler, Stickstoffeinleitungsrohr) löst man 6,4 g (0,05 mol) = 4,4 ml Oxalylchlorid in 100 ml THF und tropft bei 50 °C 9,8 g (0,05 mol) 5-Bromindol in 50 ml THF zu. DC-Kontrolle zeigt an, daß nach ca. einer Stunde die Reaktion beendet ist. Man läßt abkühlen auf Raumtemperatur und tropft unter Rühren 17,1 g (0,15 mol) = 20,4 ml cis-2,6-Dimethylpiperidin in 50 ml THF zu. Unter leichter Erwärmung verfärbt sich die Lösung hellgelb, ein farbloser Niederschlag fällt aus. Man rührt noch eine Stunde nach, bis DC-Kontrolle kein Zwischenprodukt mehr zeigt. Nach Einengen zur Trockne rührt man mit 100 ml Wasser aus. Auf dem Wasser schwimmt ein gelbes Öl, das mit dreimal 50 ml Dichlormethan extrahiert wird. Nach Trocknen mit Natriumsulfat engt man auf ein kleines Volumen ein und chromatographiert (Kieselgel 60, Laufmittel : Dichlormethan/Methanol = 20/1). Die erhaltene Hauptfraktion wird zur Trockne eingedampft und aus Essigsäureethylester rekristallisiert.
farblose Kristalle ; Schmelzpunkt : 227 °C
Ausbeute : 5,8 g (32 % der Theorie, bezogen auf 5-Bromindol).
3.2.
3-[2-(cis-2,6-Dimethylpiperidinyl)-ethyl]-5-bromindolhydrochlorid

analog 1.3. aus 2,0 g (0,055 mol) Lithiumaluminiumhydrid und 5,0 g (0,0138 mol) cis-5-Bromindolyl-3-glyoxyl-2,6-dimethylpiperidid.

Chromatographie (Kieselgel 60, Dichlormethan/Methanol = 1/1), Rekristallisation aus Diisopropylether farblose Kristalle ; Schmelzpunkt : 172 °C

Ausbeute : 2,3 g (50 % der Theorie)

Hydrochlorid

farblose Kristalle ; Fp : = 239 °C (Zers.), Ausbeute : 90 % der Theorie.

Beispiel 4

3-[2-(cis-2,6-Dimethylpiperidinyl)-ethyl]-5-chlorindol-hydrochlorid

cis

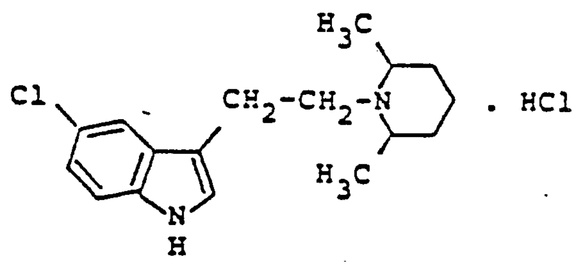

4.1.

cis-5-Chlorindolyl-3-glyoxyl-2,6-dimethylpiperidid

analog 3.1. aus 6,4 g (0,05 mol) = 4,4 ml Oxalylchlorid, 7,6 g (0,05 mol) 5-Chlorindol und 17,1 g (0,15 mol) = 20,4 ml cis-2,6-Dimethylpiperidin

Rekristallisation aus Dichlormethan

farblose Kristalle ; Schmelzpunkt : 235 °C

Ausbeute : 4,3 g (27 % der Theorie, bezogen auf 5-Chlorindol).

4.2.

3-[2-(cis-2,6-Dimethylpiperidinyl)-ethyl]-5-chlorindol-hydrochlorid

analog 1.3. aus 1,1 g (0,031 mol) Lithiumaluminiumhydrochlorid und 2,5 g (0,0078 mol) cis-5-Chlorindolyl-3-glyoxyl-2,6-dimethylpiperidid

farblose Kristalle ; Schmelzpunkt : 167 °C

Ausbeute : 1,7 g (74 % der Theorie)

Hydrochlorid

farblose Kristalle , Schmelzpunkt 240-3 °C (Zers.).

Ausbeute : 1,7 g (89 % der Theorie).

Beispiel 5

3-[2-(cis-2,6-Dimethylpiperidinyl)-ethyl]-5-fluorindol-hydrochlorid

cis

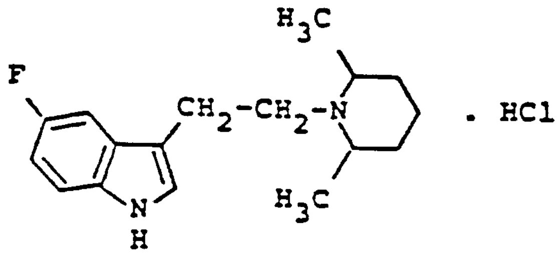

5.1.

cis-5-Fluorindolyl-3-glyoxyl-2,6-dimethylpiperidid analog 3.1. aus 4,7 g (0,037 mol) = 3,3 ml Oxalylchlorid, 5,0 g (0,037 mol) 5-Fluorindol und 12,6 g (0,111 mol) = 15 ml cis-2,6-Dimethylpiperidin

Rekristallisation aus Dichlormethan

farblose Kristalle ; Schmelzpunkt : 230 °C

Ausbeute : 4,9 g (44 % der Theorie, bezogen auf 5-Fluorindol).

5.2.

3-[2-(cis-2,6-Dimethylpiperidinyl)-ethyl]-5-fluorindol-hydrochlorid

analog 1.3. aus 2,0 g (0,053 mol) Lithiumaluminiumhydrid und 4,0 g (0,013 mol) cis-5-Fluorindolyl-3-glyoxyl-2,6-dimethylpiperidid

farblose Kristalle ; Schmelzpunkt : 182 °C

Ausbeute : 2,5 g (69 % der Theorie)

Hydrochlorid

farblose Kristalle ; Schmelzpunkt : 295 °C (Zers.)

Ausbeute : 2,1 g (93 % der Theorie).

Die Wirkung der erfindungsgemäßen Verbindungen wird im folgenden durch die Interaktion mit Serotonin-Rezeptoren beschrieben.

Beispiel 6

3-[2-(cis-2,6-Dimethylpiperidinyl)-ethyl]-5-cyanoindol-hydrochlorid

cis

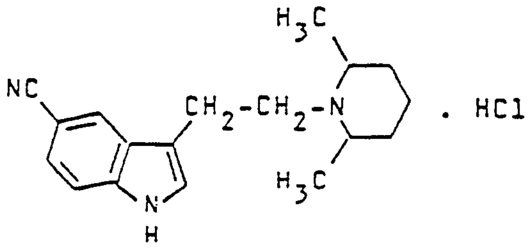

1,7 g (0,005 mol) 3-[2-(cis-2,6-Dimethylpiperidinyl)-ethyl]-5-bromindol (vgl. Beispiel 3) und 0,82 g (0,0092 mol) Kupfer(I)-cyanid werden in 10 ml N-Methyl-2-pyrrolidon suspendiert und unter Stickstoff vier Stunden zum Sieden erhitzt (Ölbad : 205-210 °C). Laut DC-Kontrolle (Laufmittel : Methanol/DMF = 1/1) ist keine Ausgangsverbindung mehr vorhanden. Man verdünnt den Ansatz mit 30 ml Methanol, filtriert vom Unlöslichen und versetzt das Filtrat mit 100 ml Wasser. Das ausgefallene Produkt wird filtriert, getrocknet und chromatographiert (Kieselgel 60, Laufmittel : ethanol/DMF = 1/1). Die geeigneten Fraktionen werden zur Trockne eingedampft und aus Diethylether rekristallisiert.

farblose Kristalle ; Schmelzpunkt : 140 °C

Ausbeute : 0,1 g (7 % der Theorie)

Hydrochlorid

farblose Kristalle ; Schmelzpunkt : 220 °C (Zers.)

(Siehe Tabelle Seite 10 f.)

Tabelle

Interaktion mit Serotonin-Rezeptoren

| Prüfsubstanz | Inhibitionskonstante $K_i$ (nmol $l^{-1}$) | | Antagonismus | |
| --- | --- | --- | --- | --- |
| | $5\text{-HT}_1$-Rezeptor Hippocampus-Kalb<br>Ligand: $^3$H-Serotonin | $5\text{-HT}_2$-Rezeptor präfrontaler Cortex-Ratte<br>Ligand: $^3$H-Ketanserin | NCB-20 Zellen Adenylatcyclase | Kontraktion A.basilaris-Hund |
| Beispiel 2 | 150 | 2500 | + | + |
| Beispiel 3 | 50 | 1300 | + | + |
| Literatur | P. Seemann et al., Eur. J. Pharmacol 66 (1980), 179-191 | J.E. Leysen et al., Mol. Pharmacol. 21 (1982), 301-314 | E. Berry-Kravis and G. Dawson, J. Neurochem. 41 (1983), 977-985 | S.J. Peroutka et al., Brain Res. 259 (1983), 327-330 |

**Patentansprüche**

1. Tryptamine der allgemeinen Formel

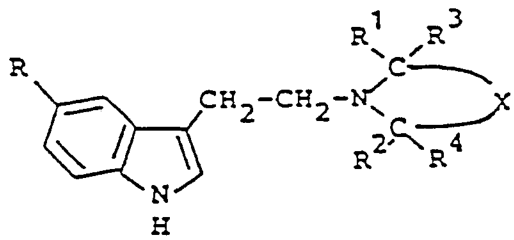

in welcher
R für Wasserstoff, Methyl, Fluor, Chlor, Brom, Cyano, Carbamoyl und Azido steht,
$R^1$ und $R^2$ gleich oder verschieden sind und Methyl oder Ethyl bedeuten,
$R^3$ und $R^4$ gleich oder verschieden sind und Wasserstoff, Methyl, Ethyl bedeuten, und
X für eine Alkylenkette mit 2 bis 4 Kohlenstoffatomen steht,
in der cis-Form, trans-Form und in Form jeglicher Gemische beider Isomere und die entsprechenden Salze.

2. Tryptamine nach Anspruch 1, in denen
R für Wasserstoff, Fluor, Chlor oder Brom steht,
$R^1$ und $R^2$ gleich oder verschieden sind und Methyl oder Ethyl bedeuten,
$R^3$ und $R^4$ Wasserstoff bedeuten, und
X für eine Alkylenkette mit 3 Kohlenstoffatomen steht.

3. Tryptamine aus der Gruppe bestehend aus

3-[2-(cis- und trans-2,5-Dimethylpyrrolinyl)-ethyl]-indol-hydrochlorid,
3-[2-(cis-2,6-Dimethylpiperidinyl)-ethyl]-indol-hydrochlorid,
3-[2-(cis-2,6-Dimethylpiperidinyl)-ethyl]-5-bromindol-hydrochlorid,
3-[2-(cis-2,6-Dimethylpiperidinyl)-ethyl]-5-chlorindol-hydrochlorid und
3-[2-(cis-2,6-Dimethylpiperidinyl)-ethyl]-5-fluorindol-hydrochlorid.

4. Tryptamine nach den Ansprüchen 1 bis 3, für eine therapeutische Behandlung.

5. Verfahren zur Herstellung von Tryptaminen der allgemeinen Formel

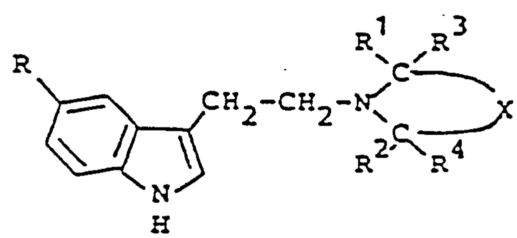

in welcher
R für Wasserstoff, Methyl, Fluor, Chlor, Brom, Cyano, Carbamoyl und Azido steht,
$R^1$ und $R^2$ gleich oder verschieden sind und Methyl oder Ethyl bedeuten,
$R^3$ und $R^4$ gleich oder verschieden sind und Wasserstoff, Methyl, Ethyl bedeuten, und
X für eine Alkylenkette mit 2 bis 4 Kohlenstoffatomen steht,
in der cis-Form, trans-Form und in Form jeglicher Gemische beider Isomere und die entsprechenden Salze, dadurch gekennzeichnet, daß man Indole der allgemeinen Formel II

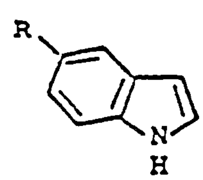

(II)

in welcher R die obengenannte Bedeutung hat, mit Oxalylchlorid zu Verbindungen der allgemeinen Formel IV

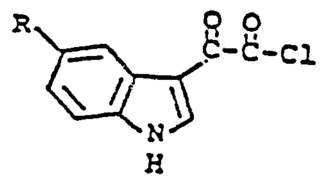

(IV)

in welcher R die obengenannte Bedeutung hat, umsetzt, die Verbindungen der Formel IV mit Aminen der Formel V

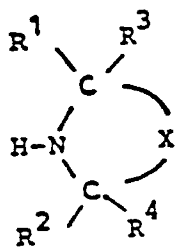

(V)

in welcher $R^1$, $R^2$, $R^3$, $R^4$ und X die obengenannte Bedeutung haben, zu Verbindungen der Formel VI

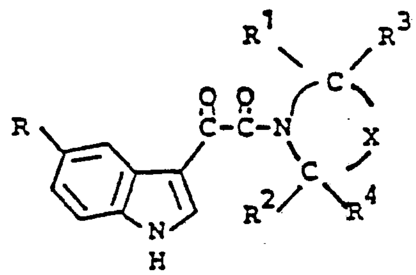

(VI)

in welcher R, $R^1$, $R^2$, $R^3$, $R^4$ und X die obengenannte Bedeutung haben, reagieren läßt und anschließend die Verbindungen der Formel VI reduziert.

6. Arzneimittel, enthaltend Tryptamine nach den Ansprüchen 1 bis 3.

7. Verwendung von Tryptaminen nach den Ansprüchen 1 bis 3 zur Herstellung von Arzneimitteln.

8. Verwendung von Tryptaminen nach Anspruch 7 zur Herstellung von Arzneimitteln zur Behandlung von Krankheiten des zentralen Nervensystems.

9. Verwendung von Tryptaminen nach den Ansprüchen 7 und 8 zur Herstellung von Arzneimitteln zur Behandlung von Schlafstörungen, Migräne, Vasospasmen und Ischämien.

10. Tryptamine nach den Ansprüchen 1 bis 3 zur Behandlung von Krankheiten des zentralen Nervensystems.

**Claims**

1. Tryptamines of the general formula

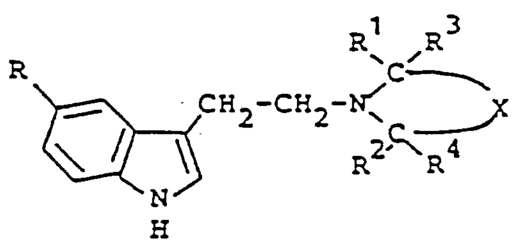

in which

R represents hydrogen, methyl, fluorine, chlorine, bromine, cyano, carbamoyl and azido,

$R^1$ and $R^2$ are identical or different and denote methyl or ethyl,

$R^3$ and $R^4$ are identical or different and denote hydrogen, methyl or ethyl, and

X represents an alkylene chain having 2 to 4 carbon atoms,

in the cis form, in the trans form, and in the form of all mixtures of the two isomers, and the corresponding salts.

2. Tryptamines according to Claim 1, in which
R represents hydrogen, fluorine, chlorine or bromine,
$R^1$ and $R^2$ are identical or different and denote methyl or ethyl,
$R^3$ and $R^4$ denote hydrogen, and X represents an alkylene chain having 3 carbon atoms.

3. Tryptamines from the group comprising

3-[2-(cis- and trans-2,5-dimethylpyrrolinyl)ethyl]indole hydrochloride,
3-[2-(cis-2,6-dimethylpiperidinyl)ethyl]indole hydrochloride,
3-[2-(cis-2,6-dimethylpiperidinyl)ethyl]-5-bromoindole hydrochloride,
3-[2-(cis-2,6-dimethylpiperidinyl)ethyl]-5-chloroindole hydrochloride and
3-[2-(cis-2,6-dimethylpiperidinyl)ethyl]-5-fluoroindole hydrochloride.

4. Tryptamines according to Claims 1 to 3, for a therapeutic treatment.

5. Process for the preparation of tryptamines of the general formula,

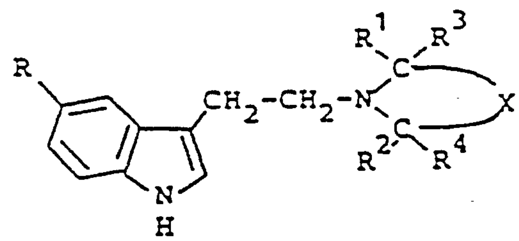

in which
R represents hydrogen, methyl, fluorine, chlorine, bromine, cyano, carbamoyl and azido,
$R^1$ and $R^2$ are identical or different and denote methyl or ethyl,
$R^3$ and $R^4$ are identical or different and denote hydrogen, methyl or ethyl, and
X represents an alkylene chain having 2 to 4 carbon atoms,
in the cis form, in the trans form, and in the form of all mixtures of the two isomers, and the corresponding salts, characterized in that indoles of the general formula II

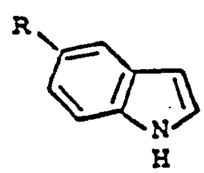

(II)

in which R has the above mentioned meaning, are reacted with oxalyl chloride to give compounds of the general formula IV

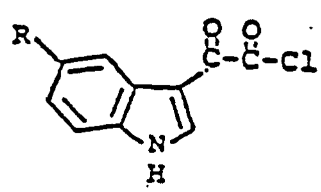

(IV)

in which R has the above-mentioned meaning, the compounds of the formula IV are allowed to react with amines of the formula V

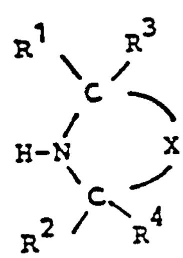

(V)

in which $R^1$, $R^2$, $R^3$, $R^4$, and X have the above-mentioned meaning, to give compounds of the formula VI

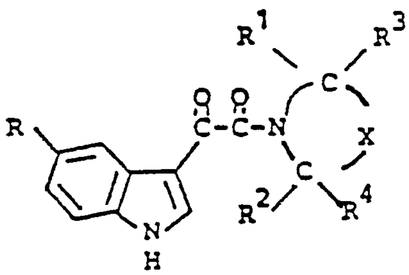

in which R, $R^1$, $R^2$, $R^3$, $R^4$, and X have the above-mentioned meaning ; and then the compounds of the formula VI are reduced.

6. Medicament containing tryptamines according to Claims 1 to 3.

7. Use of tryptamines according to Claims 1 to 3, for the preparation of medicaments.

8. Use of tryptamines according to Claim 7 for the preparation of medicaments for the treatment of diseases of the central nervous system.

9. Use of tryptamines according to Claims 7 and 8 for the preparation of medicaments for the treatment of sleep disturbances, migraine, vasospasms and ischaemias.

10. Tryptamines according to Claims 1 to 3 for the treatment of diseases of the central nervous system.

**Revendications**

1. Tryptamines de la formule générale

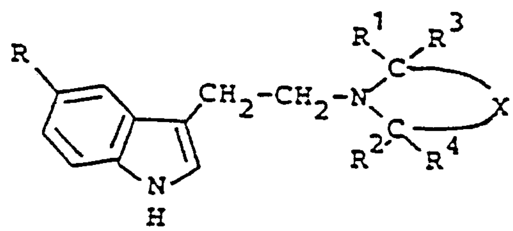

dans laquelle

R représente l'hydrogène, un radical méthyle, le fluor, le chlore, le brome, un radical cyano, carbamyle ou azido

$R^1$ et $R^2$ représentent, de manière identique ou différente, un radical méthyle ou éthyle,

$R^3$ et $R^4$ représentent, de manière identique ou différente, l'hydrogène, un radical méthyle ou éthyle, et

X représente une chaîne d'alkylène à 2 à 4 atomes de carbone,

sous forme cis, sous forme trans ou sous forme d'un mélange quelconque des deux isomères, et leurs sels correspondants.

2. Tryptamines selon la revendication 1, dans lesquelles

R représente l'hydrogène, le fluor, le chlore ou le brome,

$R^1$ et $R^2$ représentent, de manière identique ou différente, un radical méthyle ou éthyle,

$R^3$ et $R^4$ représentent l'hydrogène, et

X représente une chaîne alkylène à 3 atomes de carbone.

3. Tryptamines du groupe constitué de

chlorhydrate de 3-[2-(cis- et trans-2,5-diméthylpyrrolinyl)-éthyl]-indole,
chlorhydrate de 3-[2-(cis-2,6-diméthylpipéridinyl)-éthyl]-indole,
chlorhydrate de 3-[2-(cis-2,6-diméthylpipéridinyl)-éthyl]-5-bromo-indole,
chlorhydrate de 3-[2-(cis-2,6-diméthylpipéridinyl)-éthyl]-5-chloro-indole et
chlorhydrate de 3-[2-(cis-2,6-diméthylpipéridinyl)-éthyl]-5-fluoro-indole.

4. Tryptamines selon les revendications 1 à 3, pour un traitement thérapeutique.

5. Procédé de production de tryptamines de la formule générale

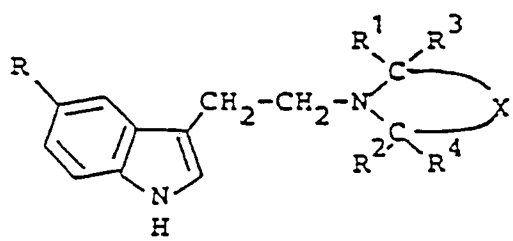

dans laquelle
R représente l'hydrogène, un radical méthyle, le fluor, le chlore, le brome, un radical cyano, carbamyle ou azido,
$R^1$ et $R^2$ représentent, de manière identique ou différente, un radical méthyle ou éthyle,
$R^3$ et $R^4$ représentent, de manière identique ou différente l'hydrogène, un radical méthyle ou éthyle, et
X représente une chaîne alkylène à 2 à 4 atomes de carbone,
sous forme cis, sous forme trans et sous forme d'un mélange quelconque des deux isomères, et des sels correspondants, caractérisé en ce que l'on fait réagir des indoles de la formule générale II

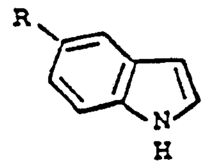

(II)

dans laquelle R a les significations précitées, avec du chlorure d'oxalyle pour obtenir des composés de la formule générale IV

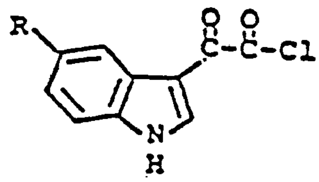

(IV)

dans laquelle R a les significations précitées, les composés de la formule IV avec des amines de la formule V

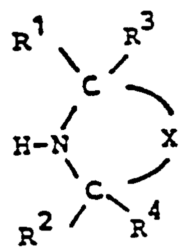

(V)

dans laquelle $R^1$, $R^2$, $R^3$ $R^4$ et X ont les significations précitées, pour obtenir des composés de la formule VI

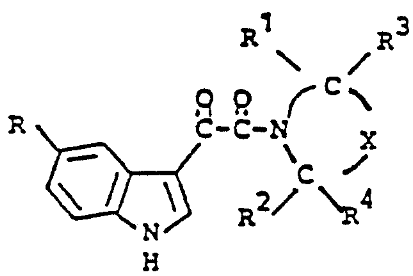

(VI)

dans laquelle R, $R^1$, $R^2$, $R^3$, $R^4$ et X ont les significations précitées, et qu'on réduit ensuite les composés de la formule VI.

6. Médicament contenant des tryptamines selon les revendications 1 à 3.

7. Utilisation des tryptamines selon les revendications 1 à 3 pour la production de médicaments.

8. Utilisation des tryptamines selon la revendication 7 pour la préparation de médicaments pour traiter les affections du système nerveux central.

9. Utilisation de tryptamines selon les revendications 7 et 8, pour la production de médicaments pour le traitement des troubles du sommeil, les migraines, les spasmes vasculaires et les ischémies.

10. Tryptamines selon les revendications 1 à 3, pour le traitement des affections du système nerveux central.